# EUROPÄISCHE PATENTSCHRIFT

(11) **EP 4 422 564 B1**
(45) Veröffentlichungstag und Bekanntmachung des Hinweises auf die Patenterteilung: **27.05.2026**
(21) Anmeldenummer: 22809664.0
(22) Anmeldetag: 21.10.2022
(51) Int. Cl.: A61F 5/01, A61F 5/02, A61F 5/05

(54) **MEHRTEILIGE STÜTZVORRICHTUNG FÜR EINE ORTHESE**
MULTI-PART SUPPORT DEVICE FOR AN ORTHOSIS
DISPOSITIF DE SUPPORT EN PLUSIEURS PARTIES POUR ORTHÈSE

(30) Priorität: 29.10.2021 DE 102021212273
(43) Veröffentlichungstag der Anmeldung: 04.09.2024
(73) Patentinhaber: Bauerfeind AG, 07937 Zeulenroda-Triebes (DE)
(72) Erfinder: PANZER, Dominique, 07937 Zeulenroda-Triebes (DE); MARX, Oliver, 07937 Zeulenroda-Triebes (DE); BAUERFEIND, Hans B., 07937 Zeulenroda-Triebes (DE)
(74) Vertreter: Wohlfahrt, Jan Günther
(86) Internationale Anmeldenummer: PCT/EP2022/079402
(87) Internationale Veröffentlichungsnummer: WO 2023/072759

(56) Entgegenhaltungen:
- EP-A1- 2 878 284
- EP-A1- 3 695 857
- EP-B1- 2 866 748
- WO-A1-2021/171252
- US-A1- 2005 165 338

## Beschreibung

Die vorliegende Erfindung betrifft orthopädische Produkte, insbesondere Orthesen und Stützvorrichtungen für Orthesen. Die Erfindung stellt eine verbesserte Stützvorrichtung für eine Orthese, ein Verfahren zu deren Herstellung und eine Orthese bereit, die eine erfindungsgemäße Stützvorrichtung enthält.

Orthesen sind therapeutische Mittel zur Stabilisierung oder Unterstützung der Bewegungsfunktion von Körperteilen. Bei der Versorgung mit funktionellen Orthesen steht die Wiederherstellung und/oder Erhaltung der Mobilität des Bewegungsapparates im Vordergrund. Orthesen können dabei funktionssicher, mobilisierend, stützend, bewegungslimitierend und/oder korrigierend auf den Bewegungsapparat wirken. Der Einsatz der Orthesen kann direkt posttraumatisch, postoperativ oder konservativ erfolgen, das heißt zum Wiederaufbau, zur Sicherung oder zum Schutz der Gelenkfunktion, zum Beispiel bei degenerativen Erkrankungen. Um die je nach Anwendung erforderliche Stabilität zu erreichen, sind in der Orthese eines oder mehrere Stabilisierungselemente vorgesehen, die in der Regel im Wesentlichen stabförmig oder schalenförmig ausgebildet sind. Die als Schalen ausgebildeten Stabilisierungselemente umgreifen das zu stabilisierende Körperteil oder Körpergelenk, beispielsweise ein Extremitätengelenk, oder den Torso im Bereich der Lendenwirbelsäure, zumeist um mehr als die Hälfte dessen Umfangs.

Die Stabilisierungselemente unterstützen die mechanisch stabilisierende Wirkung der Orthese. Orthesen mit körperteilumgreifenden Stabilisierungselementen sind beispielsweise funktionelle Orthesen für Fußgelenke, für Kniegelenke, für Hüfte und Oberschenkel, für das Handgelenk, das Ellenbogengelenk und das Schultergelenk. Solche Orthesen zeichnen sich besonders dadurch aus, dass sie das zu stabilisierende Körperteil in der Regel vollumfänglich umschließen, das heißt insbesondere zumindest mehr als die Hälfte des Umfangs des Körperteils umgreifen. Die für solche Orthesen ausgebildeten körperumgreifenden Stabilisierungselemente sind üblicherweise spangenförmig geformt und weisen dabei einen mehr als einen Halbkreis umfassenden Umfangsquerschnitt (größer als 180 Grad) um das zu umgreifende Körperteil auf.

Die Stabilisierungselemente sind in der Regel aus einem thermoplastischen oder duroplastischen Kunststoff- oder Verbundmaterial geformt. Solche nicht flächigen oder schalenförmigen und funktionsgemäß körper- oder körperteilumgreifend ausgebildeten Spangen weisen einen Hinterschnitt auf. Derartige räumlichen Strukturen sind fertigungstechnisch schwierig herzustellen. In bekannten Herstellverfahren werden körper- oder körperteilumgreifende Spangen meist aus flächigen Rohlingen (Platten) durch Thermoformung, insbesondere durch Tiefziehen, in die körper- oder körperteilumgreifende räumliche Endform (Benutzungskonformation) gebracht. Die Fertigung ist aufwändig und erfolgt in aller Regel unter hohem Material- und Personaleinsatz. In bekannter Art und Weise ausgebildete Stabilisierungsspangen für Orthesen sind alternativen und insbesondere wirtschaftlichen Fertigungstechniken nicht zugänglich. Es besteht daher der Bedarf, verbesserte Stabilisierungselemente für Orthesen bereitzustellen, die mit einfacheren und wirtschaftlicheren Fertigungstechniken hergestellt werden können und gegenüber bekannten Stabilisierungselementen zumindest gleichwertige und bevorzugt verbesserte Funktionseigenschaften aufweisen.

Im Stand der Technik ist die Kunststoff-Spritzgießtechnik zur Herstellung von Stabilisierungselementen für Orthesen bekannt. Spritzgegossene Elemente sind jedoch in der Regel im Wesentlichen flächig ausgebildete Klein- und Anbauteile oder Schalen, die in keinem Zusammenhang mit einem körperumgreifenden Stabilisierungselement stehen.

Das der Erfindung zugrundeliegende technische Problem besteht daher insbesondere darin, eine Stützvorrichtung für eine körper- oder körperteilumgreifende Orthese dergestalt weiterzubilden, dass diese einfacher und wirtschaftlicher herstellbar ist und je nach konkreter Ausgestaltung die Anwendung vereinfachen und/oder den Funktionsumfang der Orthese erweitern kann.

Das technische Problem wird durch den Gegenstand der unabhängigen Ansprüche gelöst.

Die vorliegende Erfindung betrifft dabei insbesondere eine Stützvorrichtung gemäß Anspruch 1.

Durch diese besondere Ausgestaltung der körperumgreifenden Stützvorrichtung gemäß der vorliegenden Erfindung ist es vorteilhafterweise möglich, die gewünschte Form und Funktion der Stützvorrichtung durch ein formschlüssiges Verbinden zweier Schalenelemente zu erreichen, sodass eine einfache und wirtschaftliche Herstellung der einzelnen Schalenelemente mittels Spritzgussverfahren möglich ist und gleichzeitig die Funktionalität und Stabilität einteiliger Stützvorrichtungen für Orthesen beibehalten oder verbessert wird. Besonders vorteilhaft ist hierbei, dass die Assemblierung, das heißt das formschlüssig Verbinden, der beiden Schalenelemente zum Erhalt der erfindungsgemäßen Stützvorrichtung werkzeugfrei, insbesondere mit bloßen Händen, erfolgen kann. In einer bevorzugten Ausführungsform lassen sich die mindestens zwei Rastverbindungen, durch die die beiden Schalenelemente der erfindungsgemäßen Vorrichtung miteinander verbunden werden, anschließend nicht mehr ohne erheblichen Aufwand lösen.

In besonders bevorzugter Ausführungsform der vorliegenden Erfindung sind die zwei Schalenelemente der Stützvorrichtung zueinander symmetrisch, insbesondere spiegelsymmetrisch, ausgebildet. Bevorzugt sind die zwei Schalenelemente der Stützvorrichtung zueinander im Wesentlichen symmetrisch, insbesondere im Wesentlichen spiegelsymmetrisch, ausgebildet und im Bereich der Symmetrieachse über mindestens zwei Rastverbindungen miteinander verbunden.

Erfindungsgemäß handelt es sich bei den zwei Stabilisierungselementen der erfindungsgemäßen Stützvorrichtung um zwei Schalenelemente. Erfindungsgemäß ist die Stützvorrichtung eine zwei Schalenelemente umfassende oder aus zwei Schalenelementen bestehende spangenförmige oder schalenförmige Stützvorrichtung.

Erfindungsgemäß sind die zwei Schalenelemente der Stützvorrichtung über mindestens zwei, bevorzugt mindestens drei, bevorzugt mindestens vier, Rastverbindungen formschlüssig miteinander verbunden.

Erfindungsgemäß sind die zwei Schalenelemente der Stützvorrichtung über mindestens zwei Rastverbindungen formschlüssig miteinander verbunden, wobei eine erste Rastverbindung durch Eingreifen mindestens eines Rasthakens des ersten Schalenelements in das mindestens eine Halteelement des zweiten Schalenelements ausgebildet wird und eine zweite Rastverbindung durch Eingreifen mindestens eines Rasthakens des zweiten Schalenelements in das mindestens eine Halteelement des ersten Schalenelements ausgebildet wird.

Gemäß einer weiteren bevorzugten Ausführungsform sind die zwei Schalenelemente der Stützvorrichtung über mindestens drei Rastverbindungen formschlüssig miteinander verbunden, wobei eine erste Rastverbindung durch Eingreifen mindestens eines Rasthakens des ersten Schalenelements in das mindestens eine Halteelement des zweiten Schalenelements ausgebildet wird, eine zweite Rastverbindung durch Eingreifen mindestens eines Rasthakens des zweiten Schalenelements in das mindestens eine Halteelement des ersten Schalenelements ausgebildet wird und eine dritte Rastverbindung durch Eingreifen mindestens eines Rasthakens des ersten Schalenelements in das mindestens eine Halteelement des zweiten Schalenelements ausgebildet wird. Demnach erfolgt die formschlüssige Verbindung zwischen den beiden Schalenelementen der Stützvorrichtung bevorzugt über mindestens drei Rastverbindungen, wobei die mindestens drei Rastverbindungen in alternierender Weise ausgebildet sind, das heißt, dass die erste Rastverbindung ausgebildet wird, indem mindestens ein auf dem ersten Schalenelement angeordneter Rasthaken in mindestens ein auf dem zweiten Schalenelement angeordnetes Halteelement eingreift, die zweite Rastverbindung ausgebildet wird, indem mindestens ein auf dem zweiten Schalenelement angeordneter Rasthaken in mindestens ein auf dem ersten Schalenelement angeordnetes Halteelement eingreift und die dritte Rastverbindung ausgebildet wird, indem mindestens ein auf dem ersten Schalenelement angeordneter Rasthaken in mindestens ein auf dem zweiten Schalenelement angeordnetes Halteelement eingreift. Durch diese Anordnung der Rastverbindungen zwischen den beiden Schalenelementen der Stützvorrichtung ist es vorteilhafterweise nicht möglich mit einem einzigen gerichteten Öffnungsimpuls alle Rastverbindungen gleichzeitig zu lösen. Somit wird ein unbeabsichtigtes Öffnen der Stützvorrichtung deutlich erschwert, was wiederum für zusätzliche Sicherheit für den Anwender der Stützvorrichtung sorgt.

Gemäß einer ersten Ausführungsform der vorliegenden Erfindung sind der mindestens eine Rasthaken und das mindestens eine zur formschlüssigen Aufnahme des mindestens einen Rasthakens geeignete Halteelement der mindestens einen Rastverbindung integrale Bestandteile der zwei Schalenelemente. Bevorzugt umfasst die erfindungsgemäße Stützvorrichtung neben dem ersten und dem zweiten Schalenelement keine weiteren Elemente. Gemäß dieser Ausführungsform der vorliegenden Erfindung besteht die erfindungsgemäße Stützvorrichtung somit aus dem ersten und zweiten Schalenelement, die über mindestens zwei Rastverbindungen formschlüssig miteinander verbunden sind.

Gemäß einer zweiten Ausführungsform der vorliegenden Erfindung umfasst eines der beiden Schalenelemente einen ersten Teil eines mehrteiligen Verbindungselements und das andere der beiden Schalenelemente einen zweiten Teil des mehrteiligen Verbindungselements, wobei der mindestens eine Rasthaken und das mindestens eine Halteelement der Rastverbindung Bestandteile des mehrteiligen Verbindungselements sind. Gemäß der zweiten Ausführungsform umfasst die erfindungsgemäße Stützvorrichtung somit neben dem ersten und dem zweiten Schalenelement ein mehrteiliges Verbindungselement, wobei das erste und zweite Schalenelement über mindestens zwei Rastverbindungen zwischen einem ersten Teil und einem zweiten Teil des mehrteiligen Verbindungselements miteinander verbunden sind. Gemäß dieser Ausführungsform der vorliegenden Erfindung besteht die erfindungsgemäße Stützvorrichtung aus zwei Schalenelementen und einem mehrteiligen Verbindungselement. Besonders bevorzugt umfasst die erfindungsgemäße Stützvorrichtung neben dem ersten Schalenelement, dem zweiten Schalenelement und dem mehrteiligen Verbindungselement keine weiteren Elemente.

Gemäß einer bevorzugten Ausführungsform der vorliegenden Erfindung ist das mehrteilige Verbindungselement ein zweiteiliges Verbindungselement.

In einer weiteren bevorzugten Ausführungsform der vorliegenden Erfindung ist der erste Teil des mehrteiligen Verbindungselements lösbar mit einem der zwei Schalenelemente, bevorzugt mit dem ersten Schalenelement, bevorzugt mit dem zweiten Schalenelement, verbindbar, insbesondere verbunden. Bevorzugt ist der zweite Teil des mehrteiligen Verbindungselements lösbar mit dem anderen der zwei Schalenelemente, bevorzugt mit dem ersten Schalenelement, bevorzugt mit dem zweiten Schalenelement, verbindbar, insbesondere verbunden. Besonders bevorzugt ist der erste Teil des mehrteiligen Verbindungselements lösbar mit einem der zwei Schalenelemente verbindbar, insbesondere verbunden, und der zweite Teil des mehrteiligen Verbindungselements lösbar mit dem anderen der zwei Schalenelemente verbindbar, insbesondere verbunden.

In einer bevorzugten Ausführungsform der vorliegenden Erfindung ist der erste Teil des mehrteiligen Verbindungselements unlösbar mit einem der zwei Schalenelemente, bevorzugt mit dem ersten Schalenelement, bevorzugt mit dem zweiten Schalenelement, verbindbar, insbesondere verbunden. Bevorzugt ist der zweite Teil des mehrteiligen Verbindungselements unlösbar mit dem anderen der zwei Schalenelemente, bevorzugt mit dem ersten Schalenelement, bevorzugt mit dem zweiten Schalenelement, verbindbar, insbesondere verbunden. Besonders bevorzugt ist der erste Teil des mehrteiligen Verbindungselements unlösbar mit einem der zwei Schalenelemente verbindbar, insbesondere verbunden, und der zweite Teil des mehrteiligen Verbindungselements unlösbar mit dem anderen der zwei Schalenelemente verbindbar, insbesondere verbunden.

Erfindungsgemäß ist vorgesehen, dass mindestens eine Rastverbindung durch Aufnahme mindestens eines Rasthakens des ersten Schalenelements oder eines ersten Teils eines mehrteiligen Verbindungselements in mindestens ein Halteelement des zweiten Schalenelements oder eines zweiten Teils eines mehrteiligen Verbindungselements und mindestens eine Rastverbindung durch Aufnahme mindestens eines Rasthakens des zweiten Schalenelements oder eines zweiten Teils eines mehrteiligen Verbindungselements in mindestens ein Halteelement des ersten Schalenelements oder eines ersten Teils eines mehrteiligen Verbindungselements ausgebildet wird.

Gemäß einer besonders bevorzugten Ausführungsform kann vorgesehen sein, dass die zwei Schalenelemente der Stützvorrichtung über mindestens drei Rastverbindungen zwischen einem ersten und einem zweiten Teil eines mehrteiligen Verbindungselements formschlüssig miteinander verbunden sind, wobei eine erste Rastverbindung durch Eingreifen mindestens eines Rasthakens des ersten Teils des mehrteiligen Verbindungselements in mindestens ein Halteelement des zweiten Teils des mehrteiligen Verbindungselements ausgebildet wird, eine zweite Rastverbindung durch Eingreifen mindestens eines Rasthakens des zweiten Teils des mehrteiligen Verbindungselements in mindestens ein Halteelement des ersten Teils des mehrteiligen Verbindungselements ausgebildet wird und eine dritte Rastverbindung durch Eingreifen mindestens eines Rasthakens des ersten Teils des mehrteiligen Verbindungselements in mindestens ein Halteelement des zweiten Teils des mehrteiligen Verbindungselements ausgebildet wird. Diese alternierende Anordnung der Rastverbindungen zwischen den beiden die Schalenelemente der Stützvorrichtung verbindenden Teile des mehrteiligen Verbindungselements verhindert vorteilhafterweise ein unbeabsichtigtes gleichzeitiges Öffnen aller Rastverbindungen der Stützvorrichtung durch einen einzigen gerichteten Öffnungsimpuls.

Gemäß einer besonders bevorzugten Ausführungsform der vorliegenden Erfindung umfasst der mindestens eine Rasthaken einen Rastkopf und eine Rastnase.

Bevorzugt handelt es sich bei dem mindestens einen Halteelement um eine Eingriffsöffnung mit einer Rastkante. Bevorzugt besteht die Rastkante aus einem elastischen Material.

In einer bevorzugten Ausführungsform der vorliegenden Erfindung weisen das erste und das zweite Schalenelement oder der erste und zweite Teil des mehrteiligen Verschlusselements jeweils mindestens eine Zunge auf.

Bevorzugt weisen das erste und das zweite Schalenelement oder der erste und zweite Teil des mehrteiligen Verschlusselements jeweils mindestens eine Zunge mit mindestens einem auf der Zunge angeordneten Rasthaken auf.

Gemäß einer weiteren bevorzugten Ausführungsform der vorliegenden Erfindung weisen das erste und das zweite Schalenelement oder der erste und zweite Teil des mehrteiligen Verschlusselements jeweils mindestens eine Zunge mit mindestens einem auf der Zunge angeordneten Halteelement auf.

Gemäß einer bevorzugten Ausführungsform der vorliegenden Erfindung ist der mindestens eine Rasthaken auf einer Zunge des ersten Schalenelements oder des ersten Teils des mehrteiligen Verbindungselements angeordnet. Bevorzugt ist der mindestens eine Rasthaken auf einer Zunge des zweiten Schalenelements oder des zweiten Teils des mehrteiligen Verbindungselements angeordnet.

Gemäß einer weiteren bevorzugten Ausführungsform ist das mindestens eine Halteelement auf einer Zunge des ersten Schalenelements oder des ersten Teils des mehrteiligen Verbindungselements angeordnet. Bevorzugt ist das mindestens eine Halteelement auf einer Zunge des zweiten Schalenelements oder des zweiten Teils des mehrteiligen Verbindungselements angeordnet.

Besonders bevorzugt durchdringt die Zunge im verbundenen Zustand der beiden Schalenelemente eines der beiden Schalenelemente, bevorzugt das erste Schalenelement, bevorzugt das zweite Schalenelement, oder einen der Teile des mehrteiligen Verbindungselements, bevorzugt den ersten Teil des mehrteiligen Verbindungselements, bevorzugt den zweiten Teil des mehrteiligen Verbindungselements, im Bereich einer Durchtrittsöffnung, wobei der auf der Zunge angeordnete Rasthaken rückseitig, das heißt von der Durchdringungseite abgewandten Seite, in das Halteelement des jeweils anderen Schalenelements, bevorzugt des zweiten Schalenelements, bevorzugt des ersten Schalenelements, oder des jeweils anderen Teils des mehrteiligen Verbindungselements, bevorzugt des zweiten Teils des mehrteiligen Verbindungselements, bevorzugt des ersten Teils des mehrteiligen Verbindungselements, eingreift.

Gemäß einer weiteren Ausführungsform der vorliegenden Erfindung durchdringt die Zunge im verbundenen Zustand der beiden Schalenelemente eines der beiden Schalenelemente, bevorzugt das erste Schalenelement, bevorzugt das zweite Schalenelement, oder einen der Teile des mehrteiligen Verbindungselements, bevorzugt den ersten Teil des mehrteiligen Verbindungselements, bevorzugt den zweiten Teil des mehrteiligen Verbindungselements, im Bereich einer Durchtrittsöffnung, wobei das auf der Zunge angeordnete Halteelement, den rückseitig, das heißt auf der von der Durchdringungseite abgewandten Seite, angeordneten Rasthaken eines der beiden Schalenelemente, bevorzugt des ersten Schalenelements, bevorzugt des zweiten Schalenelements, oder eines der Teile des mehrteiligen Verbindungselements, bevorzugt des ersten Teils des mehrteiligen Verbindungselements, bevorzugt des zweiten Teils des mehrteiligen Verbindungselements, aufnimmt.

In besonders bevorzugter Ausführungsform der vorliegenden Erfindung kommt es durch das Durchdringen der Zunge eines der beiden Schalenelemente, bevorzugt des ersten Schalenelements, bevorzugt des zweiten Schalenelements, oder eines der Teile des mehrteiligen Verbindungselements, bevorzugt des ersten Teils des mehrteiligen Verbindungselements, bevorzugt des zweiten Teils des mehrteiligen Verbindungselements, im Bereich einer Durchtrittsöffnung des jeweils anderen Schalenelements, bevorzugt des zweiten Schalenelements, bevorzugt des ersten Schalenelements, oder des jeweils anderen Teils des mehrteiligen Verbindungselements, bevorzugt des zweiten Teils des mehrteiligen Verbindungselements, bevorzugt des ersten Teils des mehrteiligen Verbindungselements, und das rückseitige Eingreifen des auf der Zunge angeordneten Rasthakens in das Halteelement oder die rückseitige Aufnahme des Rasthakens in das auf der Zunge angeordneten Halteelement zu einem doppelten Formschluss wodurch ein unbeabsichtigtes Öffnen der Stützvorrichtung verhindert wird.

In einer besonders bevorzugten Ausführungsform der vorliegenden Erfindung umfasst der mindestens eine Rasthaken einen Rastkopf und eine Rastnase und handelt es sich bei dem mindestens einen Halteelement um eine Eingriffsöffnung mit einer Rastkante, wobei der mindestens eine Rasthaken auf einer Zunge des ersten Schalenelements oder dem erste Teil des mehrteiligen Verbindungselements angeordnet ist, und wobei die Zunge im verbundenen Zustand der beiden Schalenelemente das zweite Schalenelement oder den zweiten Teil des mehrteiligen Verbindungselements im Bereich einer Durchtrittsöffnung durchdringt und der Rastkopf rückseitig in die Eingriffsöffnung des zweiten Schalenelements oder des zweiten Teils des mehrteiligen Verbindungselements eingreift.

Gemäß einer bevorzugten Ausführungsform der vorliegenden Erfindung kann vorgesehen sein, dass die Stützvorrichtung größenverstellbar ist.

Besonders bevorzugt wird die Größenverstellbarkeit des Umfangs der Stützvorrichtung durch das Vorliegen von mindestens zwei nebeneinander angeordneter Rasthaken und/oder mindestens zwei nebeneinander angeordneter Halteelemente der mindestens einen Rastverbindung realisiert.

Bevorzugt umfasst das erste Schalenelement oder der erste Teil des mehrteiligen Verbindungselements mindestens zwei nebeneinander angeordnete Rasthaken und/oder mindestens zwei nebeneinander angeordnete Halteelemente.

In einer weiteren bevorzugten Ausführungsform umfasst das zweite Schalenelement oder der zweite Teil des mehrteiligen Verbindungselements mindestens zwei nebeneinander angeordnete Rasthaken und/oder mindestens zwei nebeneinander angeordnete Halteelemente.

In einer besonders bevorzugten Ausführungsform umfassen das erste und zweite Schalenelement oder der erste und zweite Teil des mehrteiligen Verbindungselements das erste Schalenelement oder der erste Teil des mehrteiligen Verbindungselements jeweils mindestens zwei nebeneinander angeordnete Rasthaken und/oder mindestens zwei nebeneinander angeordnete Halteelemente.

In einer weiteren bevorzugten Ausführungsform der vorliegenden Erfindung wird die Größenverstellbarkeit des Umfangs der Stützvorrichtung durch die Austauschbarkeit des mehrteiligen Verbindungselements realisiert. Gemäß dieser bevorzugten Ausführungsform ist das mehrteilige Verbindungselement lösbar mit den zwei Schalenelementen der Stützvorrichtung verbindbar, insbesondere verbunden. Bevorzugt ist der erste Teil des mehrteilige Verbindungselements lösbar mit einem der beiden Schalenelemente verbindbar, insbesondere verbunden. Bevorzugt ist der zweite Teil des mehrteiligen Verbindungselements lösbar mit dem anderen der zwei Schalenelemente verbindbar, insbesondere verbunden. Gemäß dieser bevorzugten Ausführungsform der vorliegenden Erfindung ist eine Austauschbarkeit des mehrteiligen Verbindungselements, insbesondere des erste und/oder zweiten Teil des mehrteiligen Verbindungselements, gegeben. Dies ermöglicht beispielsweise eine Größenanpassung des Umfangs der Stützvorrichtung durch Austausch des mehrteiligen Verbindungselements, insbesondere durch des erste und/oder zweiten Teil des mehrteiligen Verbindungselements, durch ein mehrteiliges Verbindungselement, insbesondere durch einen erste und/oder zweiten Teil des mehrteiligen Verbindungselements, mit der gewünschten Dimensionierung.

Besonders bevorzugt wird die Größenverstellbarkeit der Höhe der Stützvorrichtung durch das Vorliegen von abtrennbaren Segmenten der Stützvorrichtung realisiert.

In einer besonders bevorzugten Ausführungsform sind die zwei Schalenelemente mittels Spritzgießtechnik herstellbar, insbesondere hergestellt. Bevorzugt ist das mehrteilige Verbindungselement durch Spritzgießtechnik herstellbar, insbesondere hergestellt. In einer weiteren besonders bevorzugten Ausführungsform der vorliegenden Erfindung sind die zwei Schalenelemente und das mehrteilige Verbindungselement mittels Spritzgießtechnik herstellbar, insbesondere hergestellt.

Gemäß einer weiteren besonderen Ausgestaltung der vorliegenden Erfindung ist vorgesehen, dass die Stützvorrichtung mindestens eine Durchbrechung, bevorzugt mindestens zwei Durchbrechungen, bevorzugt mindestens drei Durchbrechungen, enthält. In besonders bevorzugter Ausführungsform liegt die mindestens eine Durchbrechung im Bereich zwischen den zwei Schalenelementen der Stützvorrichtung. Bevorzugt liegt die mindestens eine Durchbrechung im Bereich zwischen dem ersten und zweiten Teil eines die zwei Schalenelemente der Stützvorrichtung verbindenden mehrteiligen Verbindungselements. In einer bevorzugten Ausführungsform hat die mindestens eine Durchbrechung eine runde Form. In einer weiteren bevorzugten Ausführungsform der vorliegenden Erfindung hat die mindestens eine Durchbrechung eine elliptische Form.

Die vorliegende Erfindung betrifft auch ein Verfahren zur Herstellung einer Stützvorrichtung für eine Orthese, enthaltend die Schritte:
a) Spritzgießen zweier Schalenelemente und
b) formschlüssiges Verbinden der beiden Schalenelemente über mindestens zwei Rastverbindungen zum Erhalt einer Stützvorrichtung für eine Orthese.

Erfindungsgemäß weist jedes der zwei Schalenelemente jeweils mindestens einen Rasthaken und mindestens ein Halteelement auf, wobei das mindestens eine Halteelement des einen der zwei Schalenelemente zur formschlüssigen Aufnahme des mindestens einen Rasthakens des anderen der zwei Schalenelemente geeignet ist, und wobei das formschlüssige Verbinden in Schritt b) über mindestens zwei Rastverbindungen durch Aufnahme des mindestens einen Rasthakens des ersten Schalenelements in das mindestens eine Halteelement des zweiten Schalenelements und durch Aufnahme des mindestens einen Rasthakens des zweiten Schalenelements in das mindestens eine Halteelement des ersten Schalenelements bewirkt wird.

In einer bevorzugten Ausführungsform der vorliegenden Erfindung weist mindestens eines der zwei Schalenelemente mindestens einen auf einer Zunge angeordneten Rasthaken auf. Bevorzugt weist mindestens eines der zwei Schalenelemente mindestens ein zur formschlüssigen Aufnahme eines Rasthakens geeignetes Halteelement auf.

Besonders bevorzugt erfolgt das formschlüssige Verbinden der beiden Schalenelemente in Schritt b) indem eine Zunge mindestens eines Schalenelements das andere Schalenelement im Bereich einer Durchtrittsöffnung durchdringt und ein auf der Zunge angeordneter Rasthaken rückseitig in ein Halteelement des anderen Schalenelements eingreift.

In einer bevorzugten Ausführungsform der vorliegenden Erfindung weist mindestens eines der zwei Schalenelemente einen auf einer Zunge angeordneten Rasthaken und das andere der zwei Schalenelemente mindestens ein zur formschlüssigen Aufnahme des Rasthakens geeignetes Halteelement auf, wobei das formschlüssige Verbinden in Schritt b) über mindestens eine Rastverbindung erfolgt und wobei die Zunge des mindestens einen Schalenelements das andere Schalenelement im Bereich einer Durchtrittsöffnung durchdringt und der auf der Zunge angeordnete Rasthaken rückseitig in das Halteelement des anderen Schalenelements eingreift.

In einer weiteren bevorzugten Ausführungsform der vorliegenden Offenbarung wird in Schritt a) mittels Spritzgießen zusätzlich ein mehrteiliges, bevorzugt zweiteiliges, Verbindungselement hergestellt, wobei ein erster Teil des Verbindungselements mit einem der zwei Schalenelemente verbindbar ist und mindestens einen Rasthaken aufweist und ein zweiter Teil des Verbindungselements mit dem anderen der zwei Schalenelemente verbindbar ist und mindestens ein Halteelement umfasst, wobei in Schritt b) ein Verbinden des ersten und zweiten Teils des mehrteiligen Verbindungselements mit dem ersten und zweiten Schalenelement erfolgt und das formschlüssige Verbinden über mindestens zwei Rastverbindungen zwischen dem ersten Teil des Verbindungselements und dem zweiten Teil des Verbindungselements jeweils durch Aufnahme des mindestens einen Rasthakens eines der Teile des Verbindungselements in das mindestens eine Halteelement des anderen Teils des Verbindungselements bewirkt wird.

Besonders bevorzugt handelt es sich bei dem erfindungsgemäßen Verfahren zur Herstellung einer Stützvorrichtung für eine Orthese, um ein Verfahren zur Herstellung einer erfindungsgemäßen Stützvorrichtung für eine Orthese.

Die vorliegende Erfindung betrifft ferner eine Orthese enthaltend eine Stützvorrichtung gemäß der vorliegenden Erfindung.

Besonders bevorzugt ist die Orthese ausgewählt aus der Gruppe bestehend aus Kniegelenkorthese, Hüftorthese, Oberschenkelorthese, Rückenorthese, insbesondere Wirbelsäulenorthese, Halskrause, Halsversteifung, Handgelenkorthese, Fußgelenkorthese, Ellenbogenorthese und Schultergelenkorthese. Bevorzugt handelt es sich bei der Orthese um eine Rückenorthese, insbesondere Wirbelsäulenorthese.

Weitere Ausführungsformen der vorliegenden Erfindung ergeben sich aus den Unteransprüchen.

Im Zusammenhang mit der vorliegenden Erfindung wird unter den Begriffen "umfassend" und "aufweisend" verstanden, dass zusätzlich zu den von diesen Begriffen explizit erfassten Elementen noch weitere, nicht explizit genannte Elemente hinzutreten können. Im Zusammenhang mit der vorliegenden Erfindung wird unter diesen Begriffen auch verstanden, dass allein die explizit genannten Elemente erfasst werden und keine weiteren Elemente vorliegen. In dieser besonderen Ausführungsform ist die Bedeutung der Begriffe "umfassend" und "aufweisend" gleichbedeutend mit dem Begriff "bestehend aus". Darüber hinaus erfassen die Begriffe "umfassend" und "aufweisend" auch Zusammensetzungen, die neben den explizit genannten Elementen auch weitere nicht genannte Elemente enthalten, die jedoch von funktionell und qualitativ untergeordneter Natur sind. In dieser Ausführungsform sind die Begriffe "umfassend" und "aufweisend" gleichbedeutend mit dem Begriff "im Wesentlichen bestehend aus".

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: a) A oder B oder C oder b) (A und B) oder c) (A und C) oder d) (B und C) oder e) (A und B und C).

Die vorliegende Erfindung wird im Folgenden anhand exemplarischer Figuren illustriert. Der Erfindungsgedanke ist nicht auf diese Ausführungsbeispiele beschränkt.

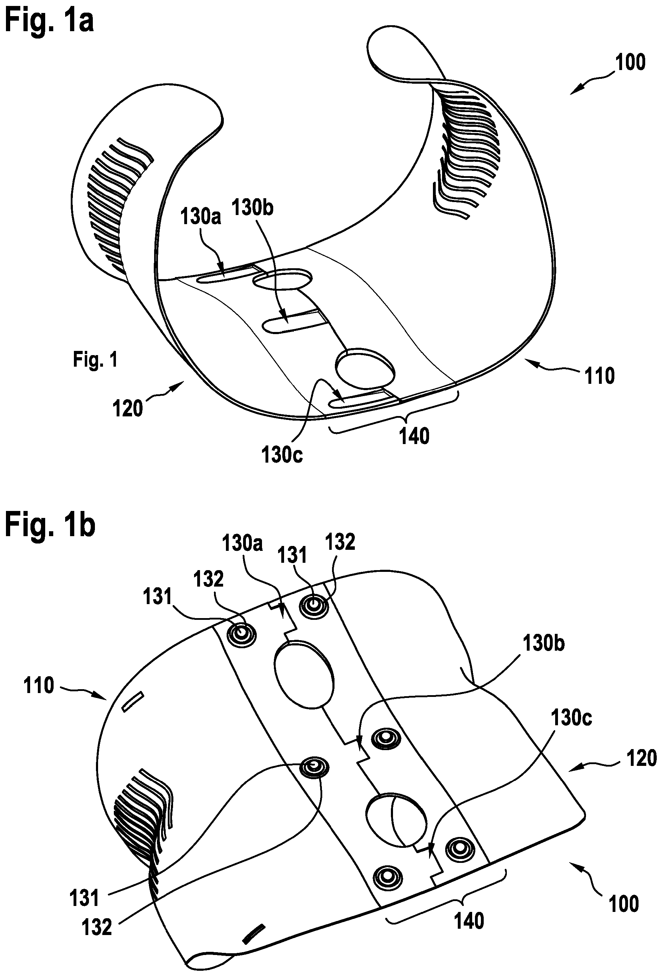

**Figur 1a** zeigt schematisch die Innenseite einer erfindungsgemäßen Stützvorrichtung (100) für eine Lumbal-Rückenorthese für den menschlichen Körper. Die Stützvorrichtung (100) der dargestellten Ausführungsform umfasst zwei Schalenelemente (110, 120) und ein mehrteiliges Verbindungselement (140), über das das erste Schalenelement (110) mittels Rastverbindungen (130a, 130b, 130c) formschlüssig mit dem zweiten Schalenelement (120) verbunden ist.

In **Figur 1b** ist schematisch die Außenseite einer erfindungsgemäßen Stützvorrichtung (100) gezeigt. In der dargestellten Ausführungsform der Erfindung werden die Rastverbindungen (130a, 130b, 130c) jeweils durch das Eingreifen eines Rasthakens (131) eines ersten Teils des mehrteiligen Verbindungselements (140) in ein Halteelement (132) eines zweiten Teils des mehrteiligen Verbindungselements (140) und das Eingreifen eines Rasthakens (131) des zweiten Teils des mehrteiligen Verbindungselements (140) in ein Halteelement (132) des ersten Teils des mehrteiligen Verbindungselements (140) ausgebildet.

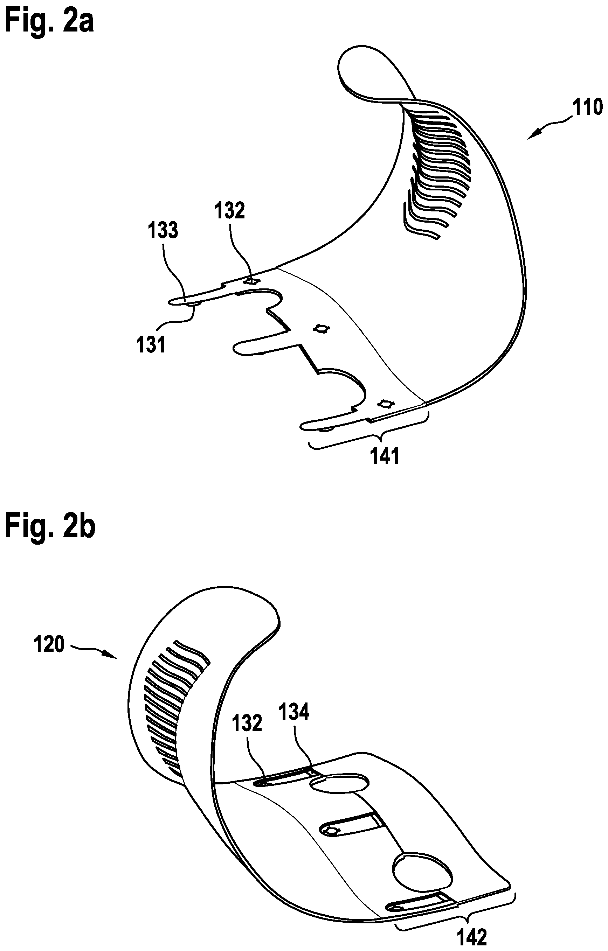

**Figur 2a** zeigt das erste Schalenelement (110) mit einem ersten Teil des mehrteiligen Verbindungselements (141) im getrennten Zustand einer erfindungsgemäßen Stützvorrichtung (100). Der erste Teil des mehrteiligen Verbindungselements (141) umfasst in der dargestellten Ausführungsform drei zur Ausbildung von Rastverbindungen geeignete Zungen (133) mit darauf angeordneten Rasthaken (131) und drei zur Aufnahme von Rasthaken (131) geeignete Halteelemente (132).

**Figur 2b** zeigt das zweite Schalenelement (120) mit einem zweiten Teil des mehrteiligen Verbindungselements (142) im getrennten Zustand einer erfindungsgemäßen Stützvorrichtung (100). Der zweite Teil des mehrteiligen Verbindungselements (142) umfasst in der dargestellten Ausführungsform drei zur Aufnahme von Rasthaken (131) geeignete Halteelemente (132), drei Durchtrittsöffnungen (134) zur Durchführung einer Zunge (133) des ersten Teils des mehrteiligen Verbindungselements (141) sowie auf der nicht dargestellten Seite des zweiten Teils des mehrteiligen Verbindungselements (142) angeordnete Rasthaken (131).

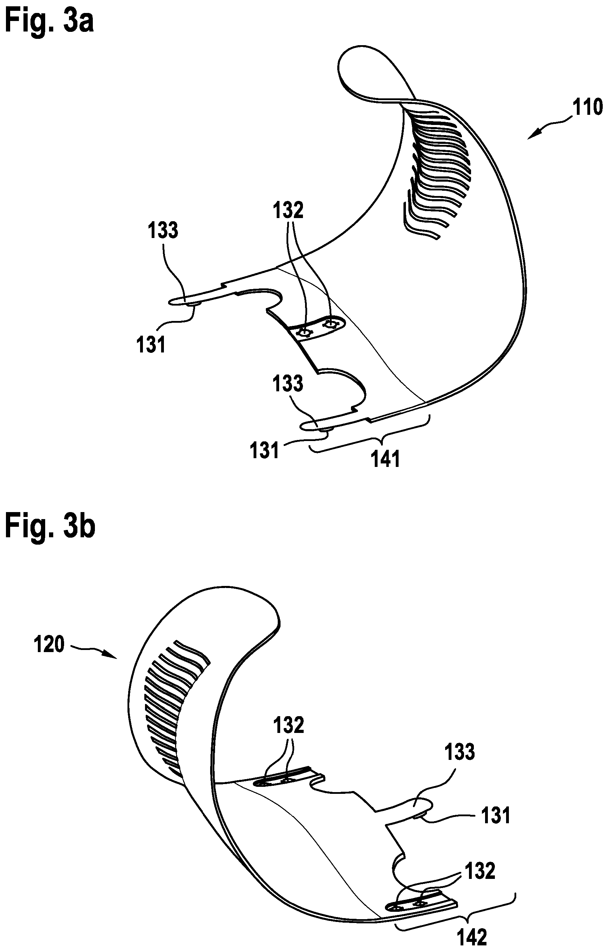

In den **Figuren 3a und 3b** ist eine besondere Ausführungsform einer erfindungsgemäßen Stützvorrichtung gezeigt, bei der das erste Schalenelement (110) einen ersten Teil des mehrteiligen Verbindungselements (141) aufweist und das zweite Schalenelement (120) einen zweiten Teil des mehrteiligen Verbindungselements (142) aufweist. Gemäß der dargestellten Ausführungsform sind die zwei Schalenelemente (110, 120) der Stützvorrichtung über drei Rastverbindungen formschlüssig miteinander verbindbar, wobei eine erste Rastverbindung durch Eingreifen eines auf einer Zunge (133) angeordneten Rasthakens (131) des ersten Teils des mehrteiligen Verbindungselements (141) in ein Halteelement (132) des zweiten Teils des mehrteiligen Verbindungselements (142) ausgebildet wird, eine zweite Rastverbindung durch Eingreifen eines auf einer Zunge (133) angeordneten Rasthakens (131) des zweiten Teils des mehrteiligen Verbindungselements (142) in ein Halteelement (132) des ersten Teils des mehrteiligen Verbindungselements (141) ausgebildet wird und eine dritte Rastverbindung durch Eingreifen eines auf einer Zunge (133) angeordneten Rasthakens (131) des ersten Teils des mehrteiligen Verbindungselements (141) in ein Halteelement (132) des zweiten Teils des mehrteiligen Verbindungselements (142) ausgebildet wird. In der dargestellten Ausführungsform weisen der erste (141) beziehungsweise zweiten Teil (142) des mehrteiligen Verbindungselements (140) zur Größenverstellung des Umfangs zwei nebeneinander angeordnete, voneinander beabstandete Halteelemente (132) auf.

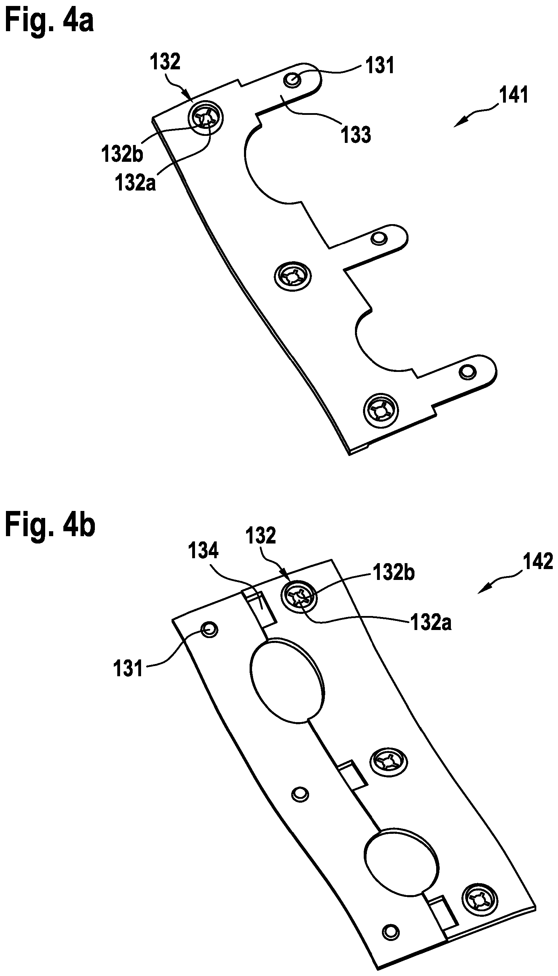

**Figur 4a** zeigt einen ersten Teil eines mehrteiligen Verbindungselements (141) umfassend einen auf einer Zunge (133) angeordneten Rasthaken (131), der zum Eingreifen in ein Halteelement (132) eines zweiten Teils eines mehrteiligen Verbindungselements (142) dient und ein Halteelement (132) mit einer Eingriffsöffnung (132a) mit einer Rastkante (132b), das zur Aufnahme eines Rasthakens (131) des zweiten Teils eines mehrteiligen Verbindungselements (142) dient.

**In** **Figur 4b** ist schematisch der zweite Teil eines des mehrteiligen Verbindungselements (142) dargestellt. Dieser umfasst einen Rasthaken (131), der zum Eingreifen in ein Halteelement (132) des ersten Teils eines mehrteiligen Verbindungselements (141) dient, ein Halteelement (132) mit einer Eingriffsöffnung (132a) mit einer Rastkante (132b), das zur Aufnahme eines Rasthakens (131) des ersten Teils eines mehrteiligen Verbindungselements (141) dient sowie eine Durchtrittsöffnung (134) durch welche eine Zunge (133) des ersten Teils des mehrteiligen Verbindungselements (141) geführt wird, sodass der auf der Zunge (133) des ersten Teils des mehrteiligen Verbindungselements (141) angeordnete Rasthaken (131) rückseitig in die Eingriffsöffnung (132a) des zweiten Teils des mehrteiligen Verbindungselements (142) eingreifen kann.

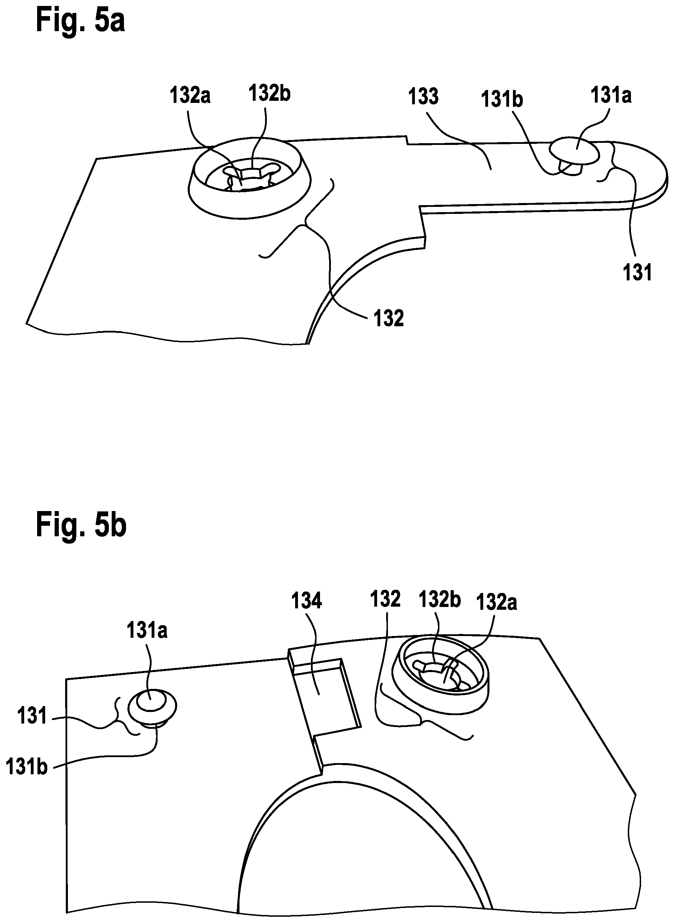

**Figur 5a** zeigt schematisch einen Ausschnitt des ersten Teils eines mehrteiligen Verbindungselements (141). Dargestellt ist ein auf einer Zunge (133) angeordneter Rasthaken (131), der zum Eingreifen in ein Halteelement (132) eines zweiten Teils eines mehrteiligen Verbindungselements (142) dient und einen Rastkopf (131a) und eine Rastnase (131b) aufweist. Ferner zeigt **Figur 5a** ein Halteelement (132) mit einer Eingriffsöffnung (132a) und einer Rastkante (132b), das zur Aufnahme eines Rasthakens (131) des zweiten Teils eines mehrteiligen Verbindungselements (142) dient.

**In** **Figur 5b** ist schematisch ein Ausschnitt des zweiten Teils eines mehrteiligen Verbindungselements (142) dargestellt. Der Ausschnitt zeigt einen Rasthaken (131), der einen Rastkopf (131a) und eine Rastnase (131b) umfasst, eine Durchtrittsöffnung (134) durch die eine Zunge (133) des ersten Teils des mehrteiligen Verbindungselements (141) geführt wird und ein Haltelement (132), das eine Eingriffsöffnung (132a) und einer Rastkante (132b) umfasst.

## Patentansprüche

1. Mehrteilige Stützvorrichtung (100) für eine Orthese, die spangenförmig oder schalenförmig ausgebildet ist, um ein Körperteil in dessen Gesamtumfang überwiegend zu umgreifen und zwei einzelne Schalenelemente (110, 120) umfasst, die geeignet sind sich jeweils über weniger als die Hälfte des Gesamtumfangs des zu umgreifenden Körpers zu erstrecken, wobei die zwei Schalenelemente (110, 120) der Stützvorrichtung (100) über mindestens zwei Rastverbindungen (130a, 130b) formschlüssig miteinander verbunden sind, **dadurch gekennzeichnet, dass** jedes der zwei Schalenelemente (110, 120) oder ein erster Teil (141) und ein zweiter Teil (142) eines zwischen den zwei Schalenelementen (110, 120) angeordneten mehrteiligen Verbindungselements (140) jeweils mindestens einen Rasthaken (131) und mindestens ein Halteelement (132) zur Ausbildung der mindestens zwei Rastverbindungen (130a, 130b) aufweist, wobei das mindestens eine Halteelement (132) eines Schalenelements (110, 120) oder eines Teils (141, 142) des mehrteiligen Verbindungselements (140) zur formschlüssigen Aufnahme des mindestens einen Rasthakens (131) der anderen Schalenelements (110, 120) oder des anderen Teils (141, 142) des mehrteiligen Verbindungselements (140) geeignet ist und wobei der mindestens eine Rasthaken (131) und das mindestens eine Halteelement (132) der Rastverbindungen (130a, 130b) integrale Bestandteile der zwei Schalenelemente (110, 120) oder des erster Teils (141) und des zweiten Teils (142) des mehrteiliges Verbindungselements (140) sind, und wobei eine erste Rastverbindung (130a) der mindestens zwei Rastverbindungen (130a, 130b) durch Eingreifen des mindestens einen Rasthakens (131) des ersten Schalenelements (110) oder des ersten Teils (141) des mehrteiliges Verbindungselements (140) in das mindestens eine Halteelement (132) des zweiten Schalenelements (120) oder des zweiten Teils (142) des mehrteiliges Verbindungselements (140) ausgebildet wird und eine zweite Rastverbindung (130b) der mindestens zwei Rastverbindungen (130a, 130b) durch Eingreifen des mindestens einen Rasthakens (131) des zweiten Schalenelements (120) oder des zweiten Teils (142) des mehrteiliges Verbindungselements (140) in das mindestens eine Halteelement (132) des ersten Schalenelements (110) oder des ersten Teils (141) des mehrteiliges Verbindungselements (140) ausgebildet wird.

2. Mehrteilige Stützvorrichtung nach Anspruch 1, **dadurch gekennzeichnet, dass** die zwei Schalenelemente (110, 120) der Stützvorrichtung (100) über mindestens drei Rastverbindungen (130a, 130b, 130c) formschlüssig miteinander verbunden sind, wobei eine erste Rastverbindung (130a) der mindestens drei Rastverbindungen (130a, 130b, 130c) durch Eingreifen des mindestens einen Rasthakens (131) des ersten Schalenelements (110) oder des ersten Teils (141) des mehrteiliges Verbindungselements (140) in das mindestens eine Halteelement (132) des zweiten Schalenelements (120) oder des zweiten Teils (142) des mehrteiliges Verbindungselements (140) ausgebildet wird, eine zweite Rastverbindung (130b) der mindestens drei Rastverbindungen (130a, 130b, 130c) durch Eingreifen des mindestens einen Rasthakens (131) des zweiten Schalenelements (120) oder des zweiten Teils (142) des mehrteiliges Verbindungselements (140) in das mindestens eine Halteelement (132) des ersten Schalenelements (110) oder des ersten Teils (141) des mehrteiliges Verbindungselements (140) ausgebildet wird und eine dritte Rastverbindung (130c) der mindestens drei Rastverbindungen (130a, 130b, 130c) durch Eingreifen eines weiteren Rasthakens (131) des ersten Schalenelements (110) oder des erster Teils (141) des mehrteiliges Verbindungselements (140) in ein weiteres Halteelement (132) des zweiten Schalenelements (120) oder des zweiten Teils (142) des mehrteiliges Verbindungselements (140) ausgebildet wird.

3. Mehrteilige Stützvorrichtung nach Anspruch 1 oder 2, **dadurch gekennzeichnet, dass** der erste Teil (141) des mehrteiligen Verbindungselements (140) lösbar mit einem der zwei Schalenelemente (110, 120) verbindbar ist und der zweite Teil (142) des mehrteiligen Verbindungselements (140) lösbar mit dem anderen der zwei Schalenelemente (110, 120) verbindbar ist.

4. Mehrteilige Stützvorrichtung nach einem der vorhergehenden Ansprüche, **dadurch gekennzeichnet, dass** der mindestens eine Rasthaken (131) einen Rastkopf (131a) und eine Rastnase (131b) umfasst und es sich bei dem mindestens einen Halteelement (132) um eine Eingriffsöffnung (132a) mit einer Rastkante (132b) handelt, wobei der mindestens eine Rasthaken (131) auf einer Zunge (133) des ersten Schalenelements (110) oder des ersten Teils (141) des mehrteiligen Verbindungselements (140) angeordnet ist, wobei die Zunge (133) im verbundenen Zustand der beiden Schalenelemente (110, 120) das zweite Schalenelement (120) oder den zweiten Teil (142) des mehrteiligen Verbindungselements (140) im Bereich einer Durchtrittsöffnung (134) durchdringt und der Rastkopf (131a) rückseitig in die Eingriffsöffnung (132a) des zweiten Schalenelements (120) oder des zweiten Teils (142) des mehrteiligen Verbindungselements (140) eingreift.

5. Mehrteilige Stützvorrichtung nach einem der vorhergehenden Ansprüche, wobei die zwei Schalenelemente (110, 120) mittels Spritzgießen herstellbar sind.

6. Verfahren zur Herstellung einer mehrteiligen Stützvorrichtung (100) für eine Orthese, enthaltend die Schritte:
a) Spritzgießen zweier Schalenelemente (110, 120), wobei jedes der zwei Schalenelemente (110, 120) jeweils mindestens einen Rasthaken (131) und mindestens ein Halteelement (132) aufweist, wobei das mindestens eine Halteelement (132) eines Schalenelements (110, 120) zur formschlüssigen Aufnahme des mindestens einen Rasthakens des anderen Schalenelements (110, 120) geeignet ist und wobei der mindestens eine Rasthaken (131) und das mindestens eine Halteelement (132) integrale Bestandteile der zwei Schalenelemente (110, 120) sind, und
b) formschlüssiges Verbinden der beiden Schalenelemente (110, 120) über mindestens zwei Rastverbindungen (130a, 130b) zum Erhalt einer spangenförmig oder schalenförmig ausgebildeten mehrteiligen Stützvorrichtung (100) für eine Orthese, wobei eine erste Rastverbindung (130a) der mindestens zwei Rastverbindungen (130a, 130b) durch Eingreifen des mindestens einen Rasthakens (131) des ersten Schalenelements (110) in das mindestens eine Halteelement (132) des zweiten Schalenelements (120) ausgebildet wird und eine zweite Rastverbindung (130b) der mindestens zwei Rastverbindungen (130a, 130b) durch Eingreifen des mindestens einen Rasthakens (131) des zweiten Schalenelements (120) in das mindestens eine Halteelement (132) des ersten Schalenelements (110) ausgebildet wird.

7. Verfahren nach Anspruch 6, wobei mindestens eines der zwei Schalenelemente (110, 120) eine Zunge (133) aufweist, auf der der mindestens eine Rasthaken (131) angeordnet ist, und das andere der zwei Schalenelemente (110, 120) eine Durchtrittsöffnung (134) aufweist, wobei die Zunge (133) des mindestens einen Schalenelements (110, 120) das andere Schalenelement (110, 120) im Bereich der Durchtrittsöffnung (134) durchdringt und der auf der Zunge (133) angeordnete Rasthaken (131) rückseitig in das Halteelement (132) des anderen Schalenelements (110, 120) eingreift.

8. Orthese, enthaltend eine mehrteilige Stützvorrichtung (100) nach einem der Ansprüche 1 bis 5.

9. Orthese nach Anspruch 8, ausgewählt aus der Gruppe bestehend aus Kniegelenkorthese, Hüftorthese, Oberschenkelorthese, Rückenorthese, insbesondere Wirbelsäulenorthese, Halskrause, Halsversteifung, Handgelenksorthese, Fußgelenksorthese, Ellenbogenorthese und Schultergelenkorthese.

## Claims

1. A multi-part support device (100) for an orthosis, which is clasp-shaped or bowl-shaped to predominantly clasp a body part along its entire circumference and which comprises two individual shell elements (110, 120), each of which is adapted to extend over less than half of the total circumference of the body part to be clasped, wherein the two shell elements (110, 120) of the support device (100) are connected to one another in a form-fitting manner via at least two snap-fit connections (130a, 130b), **characterized in that** each of the two shell elements (110, 120) or a first part (141) and a second part (142) of a multi-part connecting element (140) arranged between the two shell elements (110, 120) each comprise at least one latching hook (131) and at least one retaining element (132) for forming the at least two snap-fit connections (130a, 130b), wherein the at least one retaining element (132) of a shell element (110, 120) or a part (141, 142) of the multi-part connecting element (140) is suitable for the form-fitting reception of the at least one latching hook (131) of the other shell element (110, 120) or the other part (141, 142) of the multi-part connecting element (140), and wherein the at least one latching hook (131) and the at least one retaining element (132) of the snap-fit connections (130a, 130b) are integral components of the two shell elements (110, 120) or of the first part (141) and the second part (142) of the multi-part connecting element (140), and wherein a first snap-fit connection (130a) of the at least two snap-fit connections (130a, 130b) is formed by engagement of the at least one latching hook (131) of the first shell element (110) or of the first part (141) of the multi-part connecting element (140) in the at least one retaining element (132) of the second shell element (120) or of the second part (142) of the multi-part connecting element (140), and a second snap-fit connection (130b) of the at least two snap-fit connections (130a, 130b) is formed by engagement of the at least one latching hook (131) of the second shell element (120) or of the second part (142) of the multi-part connecting element (140) in the at least one retaining element (132) of the first shell element (110) or of the first part (141) of the multi-part connecting element (140).

2. The multi-part support device according to claim 1, **characterized in that** the two shell elements (110, 120) of the support device (100) are connected to one another in a form-fitting manner via at least three snap-fit connections (130a, 130b, 130c), wherein a first snap-fit connection (130a) of the at least three snap-fit connections (130a, 130b, 130c) is formed by engagement of the at least one latching hook (131) of the first shell element (110) or of the first part (141) of the multi-part connecting element (140) in the at least one retaining element (132) of the second shell element (120) or of the second part (142) of the multi-part connecting element (140), a second snap-fit connection (130b) of the at least three snap-fit connections (130a, 130b, 130c) is formed by engagement of the at least one latching hook (131) of the second shell element (120) or of the second part (142) of the multi-part connecting element (140) in the at least one retaining element (132) of the first shell element (110) or of the first part (141) of the multi-part connecting element (140), and a third snap-fit connection (130c) of the at least three snap-fit connections (130a, 130b, 130c) is formed by engagement of a further latching hook (131) of the first shell element (110) or of the first part (141) of the multi-part connecting element (140) in a further retaining element (132) of the second shell element (120) or of the second part (142) of the multi-part connecting element (140).

3. The multi-part support device according to claim 1 or 2, **characterized in that** the first part (141) of the multi-part connecting element (140) is detachably connectable to one of the two shell elements (110, 120), and the second part (142) of the multi-part connecting element (140) is detachably connectable to the other of the two shell elements (110, 120).

4. The multi-part support device according to any one of the preceding claims, **characterized in that** the at least one latching hook (131) comprises a latching head (131a) and a latching lug (131b), and the at least one retaining element (132) is an access opening (132a) with a latching edge (132b), wherein the at least one latching hook (131) is arranged on a tongue (133) of the first shell element (110) or of the first part (141) of the multi-part connecting element (140), wherein, when the two shell elements (110, 120) are connected, the tongue (133) penetrates the second shell element (120) or the second part (142) of the multi-part connecting element (140) in the region of a through-opening (134), and the latching head (131a) engages from the rear side in the access opening (132a) of the second shell element (120) or of the second part (142) of the multi-part connecting element (140).

5. The multi-part support device according to any one of the preceding claims, wherein the two shell elements (110, 120) can be manufactured by injection molding.

6. A method for manufacturing a multi-part support device (100) for an orthosis, comprising the steps:
a) injection molding two shell elements (110, 120), wherein each of the two shell elements (110, 120) has at least one latching hook (131) and at least one retaining element (132), wherein the at least one retaining element (132) of a shell element (110, 120) is adapted to receive the at least one latching hook (131) of the other shell element (110, 120) in a form fitting manner, and wherein the at least one latching hook (131) and the at least one retaining element (132) are integral components of the two shell elements (110, 120), and
b) connecting the two shell elements (110, 120) in a form fitting manner via at least two snap-fit connections (130a, 130b) to form a clasp-shaped or bowl-shaped multi-part support device (100) for an orthosis, wherein a first snap-fit connection (130a) of the two snap-fit connections (130a, 130b) is formed by engagement of the at least one latching hook (131) of the first shell element (110) in the at least one retaining element (132) of the second shell element (120), and a second snap-fit connection (130b) of the two snap-fit connections (130a, 130b) is formed by engagement of the at least one latching hook (131) of the second shell element (120) in the at least one retaining element (132) of the first shell element (110).

7. The method according to claim 6, wherein at least one of the two shell elements (110, 120) has a tongue (133) on which the at least one latching hook (131) is arranged, and the other of the two shell elements (110, 120) comprises a through-opening (134), wherein the tongue (133) of the at least one shell element (110, 120) penetrates the other shell element (110, 120) in the region of the through-opening (134), and the latching hook (131) arranged on the tongue (133) engages from the rear side in the retaining element (132) of the other shell element (110, 120).

8. An orthosis comprising a multi-part support device (100) according to any one of claims 1 to 5.

9. The orthosis according to claim 8, selected from the group consisting of a knee joint orthosis, a hip orthosis, a thigh orthosis, a back orthosis, in particular a spinal orthosis, a neck brace, a cervical collar, a wrist orthosis, an ankle orthosis, an elbow orthosis, and a shoulder joint orthosis.

## Revendications

1. Dispositif de soutien en plusieurs parties (100) destiné à une orthèse, réalisé en forme de bracelet ou de coque afin d'entourer principalement la circonférence totale d'une partie du corps, et comprenant deux éléments de coque (110, 120) individuels appropriés pour s'étendre respectivement sur moins de la moitié de la circonférence totale du corps à entourer, dans lequel les deux éléments de coque (110, 120) du dispositif de soutien (100) sont reliés l'un à l'autre avec verrouillage par complémentarité de forme par l'intermédiaire d'au moins deux liaisons par encliquetage (130a, 130b), **caractérisé en ce que** chacun des deux éléments de coque (110, 120) ou une première partie (141) et une deuxième partie (142) d'un élément de liaison en plusieurs parties (140) agencé entre les deux éléments de coque (110, 120) présente(nt) respectivement au moins un crochet d'encliquetage (131) et au moins un élément de retenue (132) afin de former les au moins deux liaisons par encliquetage (130a, 130b), dans lequel le au moins un élément de retenue (132) d'un élément de coque (110, 120) ou d'une partie (141, 142) de l'élément de liaison en plusieurs parties est approprié pour accueillir avec verrouillage par complémentarité de forme le au moins un crochet d'encliquetage (131) de l'autre élément de coque (110, 120) ou de l'autre partie (141, 142) de l'élément de liaison en plusieurs parties (140) et dans lequel le au moins un crochet d'encliquetage (131) et le au moins un élément de retenue (132) des liaisons par encliquetage (130a, 130b) font partie intégrante des deux éléments de coque (110, 120) ou de la première partie (141) et de la deuxième partie (142) de l'élément de liaison en plusieurs parties (140), et dans lequel une première liaison par encliquetage (130a) parmi les au moins deux liaisons par encliquetage (130a, 130b) est formée grâce à la mise en prise du au moins un crochet d'encliquetage (131) du premier élément de coque (110) ou de la première partie de l'élément de liaison en plusieurs parties (140) dans le au moins un élément de retenue (132) du deuxième élément de coque (120) ou de la deuxième partie (142) de l'élément de liaison en plusieurs parties (140), et une deuxième liaison par encliquetage (130b) parmi les au moins deux liaisons par encliquetage (130a, 130b) est formée grâce à la mise en prise du au moins un crochet d'encliquetage (131) du deuxième élément de coque (120) ou de la deuxième partie (142) de l'élément de liaison en plusieurs parties (140) dans le au moins un élément de retenue (132) du premier élément de coque (110) ou de la première partie (141) de l'élément de liaison en plusieurs parties (140).

2. Dispositif de soutien en plusieurs parties selon la revendication 1, **caractérisé en ce que** les deux éléments de coque (110, 120) du dispositif de soutien (100) sont reliés l'un à l'autre avec verrouillage par complémentarité de forme par l'intermédiaire d'au moins trois liaisons par encliquetage (130a, 130b, 130c), dans lequel une première liaison par encliquetage (130a) parmi les au moins trois liaisons par encliquetage (130a, 130b, 130c) est formée grâce à la mise en prise du au moins un crochet d'encliquetage (131) du premier élément de coque (110) ou de la première partie (141) de l'élément de liaison en plusieurs parties (140) dans le au moins un élément de retenue (132) du deuxième élément de coque (120) ou de la deuxième partie (142) de l'élément de liaison en plusieurs parties (140), une deuxième liaison par encliquetage (130b) parmi les au moins trois liaisons par encliquetage (130a, 130b, 130c) est formée grâce à la mise en prise du au moins un crochet d'encliquetage (131) du deuxième élément de coque (120) ou de la deuxième partie (142) de l'élément de liaison en plusieurs parties (140) dans au moins un élément de retenue (132) du premier élément de coque (110) ou de la première partie (141) de l'élément de liaison en plusieurs parties (140) et une troisième liaison par encliquetage (130c) parmi les au moins trois liaisons par encliquetage (130a, 130b, 130c) est formée grâce à la mise en prise d'un autre crochet d'encliquetage (131) du premier élément de coque (110) ou de la première partie (141) de l'élément de liaison en plusieurs parties (140) dans un autre élément de retenue (132) du deuxième élément de coque (120) ou de la deuxième partie (142) de l'élément de liaison en plusieurs parties (140).

3. Dispositif de soutien en plusieurs parties selon la revendication 1 ou 2, **caractérisé en ce que** la première partie (141) de l'élément de liaison en plusieurs parties (140) peut être reliée de manière amovible à l'un des deux éléments de coque (110, 120) et la seconde partie (142) de l'élément de liaison en plusieurs parties (140) peut être reliée de manière amovible à l'autre des deux éléments de coque (110, 120).

4. Dispositif de soutien en plusieurs parties selon l'une quelconque des revendications précédentes, **caractérisé en ce que** le au moins un crochet d'encliquetage (131) comprend une tête d'encliquetage (131a) et un ergot d'encliquetage (131b) et **en ce que** le au moins un élément de retenue (132) est un orifice d'encliquetage (132a) comprenant un bord d'encliquetage (132b), dans lequel le au moins un crochet d'encliquetage (131) est agencé sur une languette (133) du premier élément de coque (110) ou de la première partie (141) de l'élément de liaison en plusieurs parties (140), dans lequel la languette (133), dans l'état relié des deux éléments de coque (110, 120), traverse le deuxième élément de coque (120) ou la deuxième partie (142) de l'élément de liaison en plusieurs parties (140) dans la région d'un orifice de passage (134) et la tête d'encliquetage (131a) vient en prise par l'arrière dans l'orifice d'encliquetage (132a) du deuxième élément de coque (120) ou de la deuxième partie (142) de l'élément de liaison en plusieurs parties (140).

5. Dispositif de soutien en plusieurs parties selon l'une quelconque des revendications précédentes, dans lequel les deux éléments de coque (110, 120) peuvent être fabriqués par moulage par injection.

6. Procédé de fabrication d'un dispositif de soutien en plusieurs parties (100) destiné à une orthèse, comprenant les étapes consistant à :
a) mouler par injection deux éléments de coque (110, 120), dans lequel chacun des deux éléments de coque (110, 120) présente respectivement au moins un crochet d'encliquetage (131) et au moins un élément de retenue (132), dans lequel le au moins un élément de retenue (132) d'un élément de coque (110, 120) est approprié pour accueillir avec verrouillage par complémentarité de forme le au moins un crochet d'encliquetage de l'autre élément de coque (110, 120) et dans lequel le au moins un crochet d'encliquetage (131) et le au moins un élément de retenue (132) font partie intégrante des deux éléments de coque (110, 120), et
b) relier avec verrouillage par complémentarité de forme les deux éléments de coque (110, 120) par l'intermédiaire d'au moins deux liaisons par encliquetage (130a, 130b) afin d'obtenir un dispositif de soutien en plusieurs parties (100) en forme de bracelet ou de coque destiné à une orthèse, dans lequel une première liaison par encliquetage (130a) parmi les au moins deux liaisons par encliquetage (130a, 130b) est formée grâce à la mise en prise du au moins un crochet d'encliquetage (131) du premier élément de coque (110) dans le au moins un élément de retenue (132) du deuxième élément de coque (120) et une deuxième liaison par encliquetage (130b) parmi les au moins deux liaisons par encliquetage (130a, 130b) est formée grâce à la mise en prise du au moins un crochet d'encliquetage (131) du deuxième élément de coque (120) dans le au moins un élément de retenue (132) du premier élément de coque (110).

7. Procédé selon la revendication 6, dans lequel au moins un des deux éléments de coque (110, 120) présente une languette (133) sur laquelle est agencé le au moins un crochet d'encliquetage (131) et l'autre des deux éléments de coque (110, 120) présente un orifice de passage (134), dans lequel la languette (133) du au moins un élément de coque (110, 120) traverse l'autre élément de coque (110, 120) dans la région de l'orifice de passage (134) et le crochet d'encliquetage (131) agencé sur la languette (133) vient en prise par l'arrière dans l'élément de retenue (132) de l'autre élément de coque (110, 120).

8. Orthèse contenant un dispositif de soutien en plusieurs parties (100) selon l'une quelconque des revendications 1 à 5.

9. Orthèse selon la revendication 8, choisie dans le groupe constitué d'une orthèse de genou, d'une orthèse de hanche, d'une orthèse de cuisse, d'une orthèse du dos, en particulier d'une orthèse de colonne vertébrale, d'une minerve, d'un collier cervical, d'une orthèse de poignet, d'une orthèse de cheville, d'une orthèse de coude et d'une orthèse d'épaule.
